# EUROPEAN PATENT SPECIFICATION

(11) **EP 3 988 063 B1**
(45) Date of publication and mention of the grant of the patent: **21.06.2023**
(21) Application number: 21203866.5
(22) Date of filing: 21.10.2021
(51) Int. Cl.: A61F 9/008

(54) **OPHTHALMOLOGICAL DEVICE FOR SURGICAL TREATMENT OF A CORNEA**
OPHTHALMOLOGISCHE VORRICHTUNG ZUR CHIRURGISCHEN BEHANDLUNG EINER HORNHAUT
DISPOSITIF OPHTALMOLOGIQUE POUR LE TRAITEMENT CHIRURGICAL D'UNE CORNÉE

(30) Priority: 26.10.2020 CH 13732020
(43) Date of publication of application: 27.04.2022
(73) Proprietor: Ziemer Ophthalmic Systems AG, 2562 Port (CH)
(72) Inventor: STEINLECHNER, Michael, 8006 Zürich (CH); BERNAU, Werner, 3098 Köniz (CH)
(74) Representative: Rentsch Partner AG

(56) References cited:
- WO-A1-2015/051832
- US-A1- 2004 243 111
- US-A1- 2004 243 112
- US-B2- 10 058 453

## Description

### Field of the Disclosure

The present disclosure relates to an ophthalmological device for surgical treatment of a cornea of an eye. In particular, the present disclosure relates to an ophthalmological device for surgical treatment of a cornea of an eye using a pulsed laser beam, and to a computer program product with computer program code for controlling a processor of the ophthalmological device.

### Background of the Disclosure

For the purposes of working on eye tissue by means of a laser beam, a work region is scanned by laser pulses by virtue of the pulsed laser beam being deflected in one or more scan directions by means of suitable scanner systems (deflection apparatuses). In general, movable mirrors are used to deflect the light beams and/or the laser pulses, for example femtosecond laser pulses, said movable mirrors being pivotable about one or two scan axes, for example by way of galvano scanners, piezo scanners, polygon scanners or resonance scanners.

US 7,621,637 describes an apparatus for working on eye tissue, said apparatus having a base station with a laser source for producing laser pulses and a scanner, arranged in the base station, with movable deflection mirrors for deflecting the laser pulses in a scan direction. The deflected laser pulses are transferred via an optical relay system from the base station to an application head, the latter passing over a work region according to a scan pattern by means of a mechanically moved projection optical unit. According to US 7,621,637, in the application head, the deflection in the scan direction, which is much faster in comparison with the mechanical movement, is overlaid onto the mechanical movement of the projection optical unit and consequently onto the scan pattern thereof. A fast scanner system in the base station facilitates a fine movement of the laser pulses (micro-scan), which is overlaid on the scan pattern of the movable projection optical unit that covers a large work region, for example the entire eye.

For refractive correction, pulsed laser radiation is used in corneal surgery to create a lenticule in the cornea.

US 2016/0089270 describes a system and a method for cutting lenticules in the eye tissue. According to US 2016/0089270, straight-lined fast scan lines are overlaid to this end on slower work lines that are traced out along meridians of the lenticule.

US10058453 describes a method of corneal surgery for refractive correction using pulsed laser radiation to create a lenticule in the cornea of an eye.

To achieve the refractive correction, the created lenticule is removed from the cornea through a cut access channel. Typically, after their creation, the posterior and anterior surfaces of the lenticule are still partially attached to cornea tissue and must be separated by means of a surgical tool, particularly by means of a dissector, before the lenticule can be removed.

For removing the lenticule from the cornea tissue, US10058453 discloses creating two access channels: a straight posterior channel, which is substantially tangential to the posterior incision of the lenticule to facilitate separation of the posterior side of the lenticule from the eye, and a straight anterior channel, which is substantially tangential to the anterior incision of the lenticule to facilitate separation of the anterior side of the lenticule from the eye. One of the two channels is created with different widths at each end. The two channels are located in different circular sectors of the cornea and make it possible for the operating surgeon to access selectively the posterior side and the anterior side of the lenticule for separating and removing the lenticule from the cornea. However, at least some surgeons avoid creating the two access channels as taught by US10058453 because they fear that creating the two access channels as taught by US1 0058453 weakens the integrity of the cornea.

### Summary of the Disclosure

It is an object of the present disclosure to propose an ophthalmological device for surgical treatment of a cornea of an eye using a pulsed laser beam, which does not have at least some of the disadvantages of the prior art. Particularly, it is an object of the present disclosure to propose an ophthalmological device for surgical treatment of a cornea of an eye using a pulsed laser beam, which enables removal of a lenticule cut in the cornea with reduced impairment of the cornea.

According to the present disclosure, these objects are achieved by the features of the independent claims. Moreover, further advantageous embodiments emerge from the dependent claims and the description.

An ophthalmological device for surgical treatment of a cornea of an eye comprises: a laser source configured to generate a pulsed laser beam; a focusing optical module configured to make the pulsed laser beam converge onto a focus in the cornea; a scanner system configured to move the focus to target locations in the cornea; and an electronic circuit configured to control the scanner system to move the focus to cut inside the cornea a lenticule, the lenticule having a posterior lenticule surface and an anterior lenticule surface.

According to the present disclosure, the above-mentioned objects are particularly achieved in that the electronic circuit is further configured to control the scanner system to move the focus to cut in the cornea at least one opening incision in an exterior surface of the cornea, a first access channel, and a second access channel, the first access channel connecting the at least one opening incision to the posterior lenticule surface to provide access for a surgical tool through the at least one opening incision to the posterior lenticule surface, and the second access channel connecting the at least one opening incision to the anterior lenticule surface to provide access for the surgical tool through the at least one opening incision to the anterior lenticule surface, whereby the first access channel and the second access channel overlap at least partially from a top view perspective onto the cornea.

Configuring the electronic circuit to control the scanner system to move the focus to cut in the cornea two at least partially overlapping access channel has the advantage that the posterior and anterior surfaces of the lenticule can be completely separated from the cornea tissue by the surgeon, while weakening of the integrity of the cornea can be kept minimal as the access channels and particularly the opening incision(s) are arranged in the same circular sector of the cornea.

In an embodiment, the electronic circuit is configured to control the scanner system to move the focus to cut one common opening incision in the exterior surface of the cornea for the first access channel and the second access channel, to provide access for the surgical tool through the common opening incision and the first access channel to the posterior lenticule surface, and through the common opening incision and the second access channel to the anterior lenticule surface.

In an embodiment, the electronic circuit is further configured to control the scanner system to move the focus to cut the first access channel and the second access channel with a partially common access channel to provide access for the surgical tool through the common opening incision, the common access channel and the first access channel to the posterior lenticule surface, and through the common opening incision, the common access channel and the second access channel to the anterior lenticule surface.

In an embodiment, the electronic circuit is configured to control the scanner system to move the focus to cut in the cornea a first opening incision in the exterior surface of the cornea for the first access channel to provide access for the surgical tool through the first opening incision and the first access channel to the posterior lenticule surface, and a separate second opening incision in the exterior surface of the cornea for the second access channel to provide access for the surgical tool through the second opening incision and the second access channel to the anterior lenticule surface.

In an embodiment, the electronic circuit is configured to control the scanner system to move the focus to cut the first access channel in the cornea with a first width, and to cut the second access channel in the cornea with a second width different from the first width.

In an embodiment, the electronic circuit is configured to control the scanner system to move the focus to cut the first access channel and the second access channel in the cornea with a partial overlap from the top view perspective onto the cornea.

In an embodiment, the electronic circuit is configured to control the scanner system to move the focus to cut the first access channel in the cornea with a first symmetry plane, and to cut the second access channel in the cornea with a second symmetry plane, whereby the first symmetry plane and the second symmetry plane run parallel to each other.

In an embodiment, the electronic circuit is configured to control the scanner system to move the focus to cut the first access channel in the cornea with a first symmetry plane, and to cut the second access channel in the cornea with a second symmetry plane, whereby the first symmetry plane and the second symmetry plane run at an angle to each other.

In an embodiment, the electronic circuit is configured to control the scanner system to move the focus to cut the first access channel and the second access channel with a trapezoid shaped outline from the top view perspective onto the cornea.

In an embodiment, the electronic circuit is configured to control the scanner system to move the focus to cut at least one of the first access channel or the second access channel in the cornea from with an increasing width from the opening incision in the exterior surface of the cornea towards the posterior lenticule surface or the anterior lenticule surface, respectively.

In an embodiment, the electronic circuit is configured to control the scanner system to move the focus to cut the first access channel along a first channel axis having at least one change of direction from the opening incision in the exterior surface of the cornea to the posterior lenticule surface, and to cut the second access channel along a second channel axis having at least one change of direction from the opening incision in the exterior surface of the cornea to the anterior lenticule surface.

In an embodiment, the electronic circuit is configured to control the scanner system to move the focus to cut the first access channel to approach tangentially the posterior lenticule surface, and to cut the second access channel to approach tangentially the anterior lenticule surface.

In an embodiment, the electronic circuit is configured to control the scanner system to move the focus to cut the first access channel with a first entry channel, from the at least one opening incision in the exterior surface of the cornea to a first turning area inside the cornea, and a first connecting channel from the first turning area to the posterior lenticule surface, and to cut the second access channel with a second entry channel from the at least one opening incision in the exterior surface of the cornea to a second turning area inside the cornea, and a second connecting channel from the second turning area to the anterior lenticule surface.

In an embodiment, the electronic circuit is configured to control the scanner system to move the focus to cut the first entry channel and the second entry channel with a common channel portion from the at least one opening incision in the exterior surface of the cornea to a forking area inside the cornea.

In an embodiment, the electronic circuit is configured to control the scanner system to move the focus to cut the first entry channel and the second entry channel with different geometric channel characteristics. The different geometric channel characteristics enable the surgeon to distinguish the two access channels in a tactile fashion by inserting a surgical tool alternatingly in the access channels. For example, the different geometric channel characteristics comprise different channel widths from top view perspective, different angular arrangement of the access channels from the top view perspective, and/or different channel positions from top view perspective, while the partial overlap of the two access channels from top view perspective is maintained.

In addition to the ophthalmological device for surgical treatment of a cornea of an eye, the present disclosure further relates to a computer program product, particularly, a computer program product comprising a non-transitory computer-readable medium having stored thereon computer program code for controlling a processor of an ophthalmological device which comprises a laser source configured to generate a pulsed laser beam, a focusing optical module configured to make the pulsed laser beam converge onto a focus in the cornea, and a scanner system configured to move the focus to target locations in the cornea. The computer program code is configured to control the processor such that the processor: directs the scanner system to move the focus to cut inside the cornea a lenticule, the lenticule having a posterior lenticule surface and an anterior lenticule surface, and to move the focus according to cut in the cornea at least one opening incision, a first access channel, and a second access channel, the first access channel connecting the at least one opening incision to the posterior lenticule surface to provide access for a surgical tool through the at least one opening incision to the posterior lenticule surface, and the second access channel connecting the at least one opening incision to the anterior lenticule surface to provide access for the surgical tool through the at least one opening incision to the anterior lenticule surface, whereby the first access channel and the second access channel overlap at least partially from a top view perspective onto the cornea.

### Brief Description of the Drawings

The present disclosure will be explained in more detail, by way of example, with reference to the drawings in which:

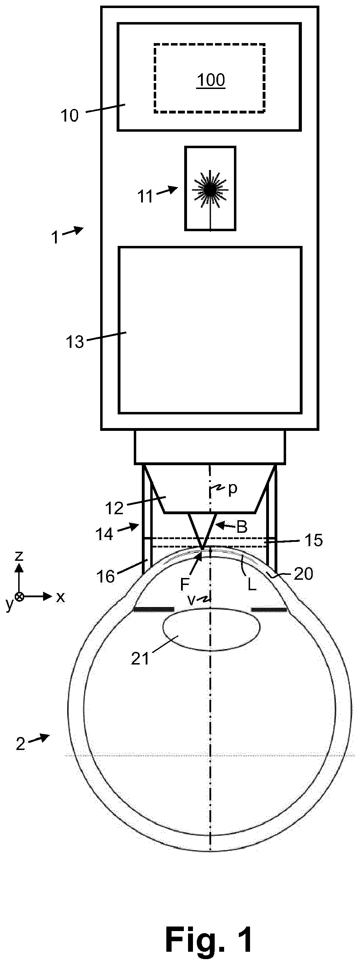

- Figure 1:: shows a block diagram that schematically illustrates an ophthalmological device for surgical treatment of a cornea with a pulsed laser beam, said device comprising a focusing optical module for focusing the pulsed laser beam in the cornea, and a scanner system for moving the focus to target locations in the cornea.

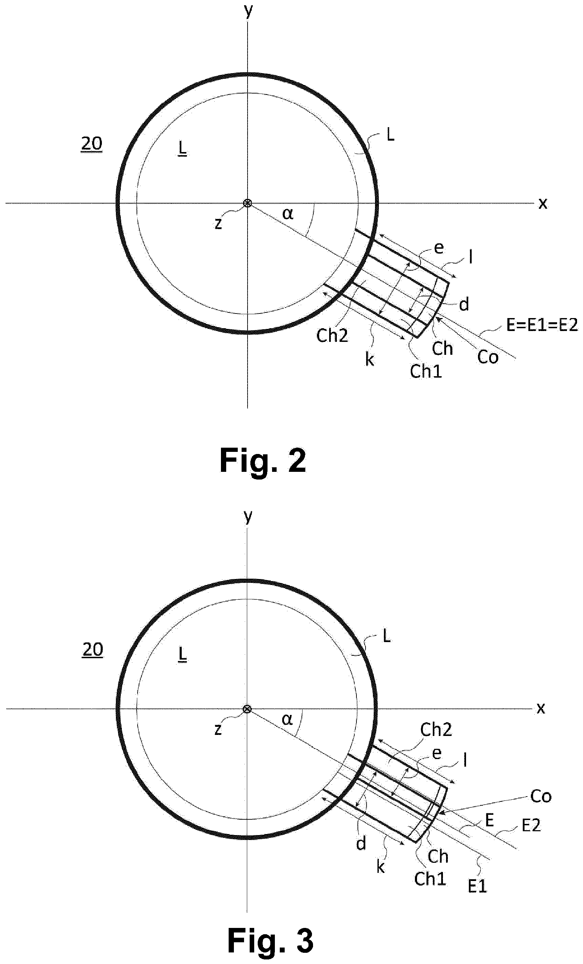

- Figures 2-6:: show schematic top views of a cornea with a lenticule cut in the cornea, and a first access channel and a second access channel cut in the cornea and providing access through one or more incisions in the cornea to the posterior lenticule surface and the anterior lenticule surface, respectively, whereby, from the top view perspective, the first access channel and the second access channel overlap at least partially.

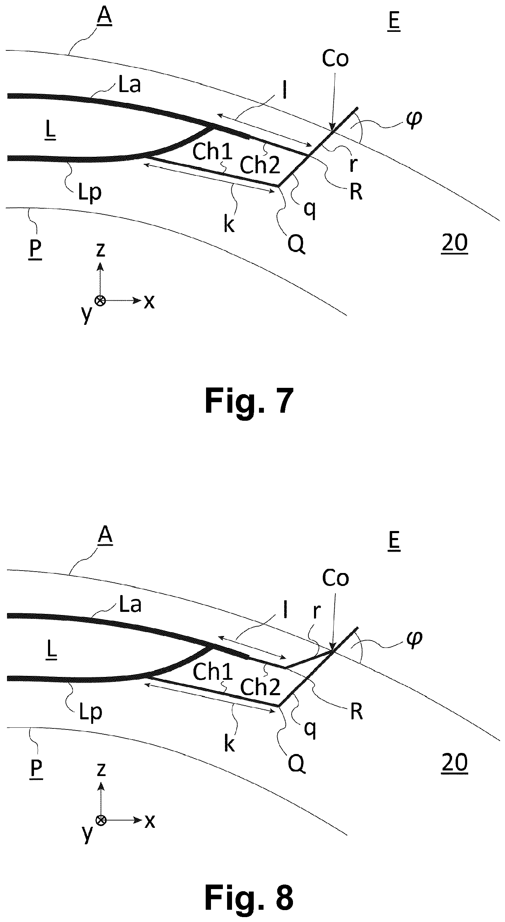

- Figures 7-9:: show schematic cross-sectional views of a section of a cornea with a lenticule cut in the cornea, and a first access channel and a second access channel cut in the cornea and providing access through a common incision in the cornea to the posterior lenticule surface and the anterior lenticule surface, respectively, whereby, in direction from the anterior surface of the cornea to the posterior surface of the cornea, the first access channel and the second access channel overlap at least partially.

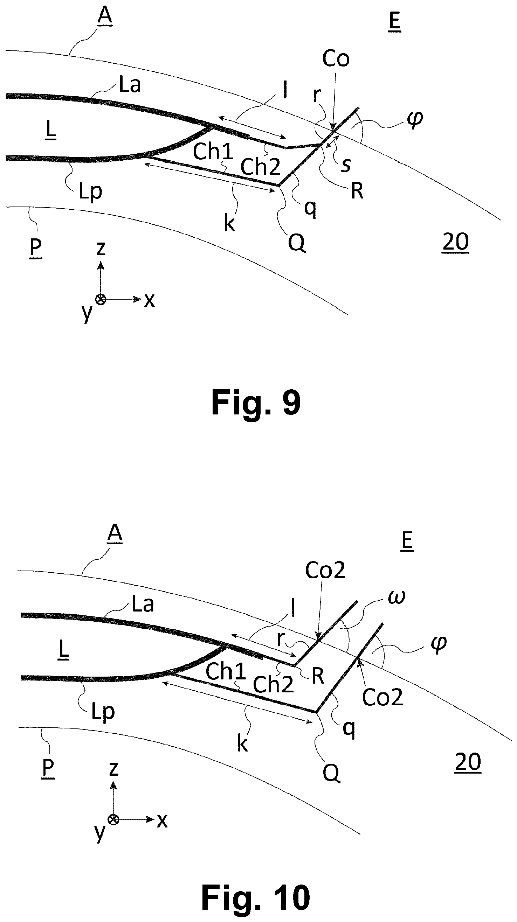

- Figure 10:: shows a schematic cross-sectional view of a section of a cornea with a lenticule cut in the cornea, a first access channel and a second access channel cut in the cornea and providing access through separate incisions in the cornea to the posterior lenticule surface and the anterior lenticule surface, respectively, whereby, in direction from the anterior surface of the cornea to the posterior surface of the cornea, the first access channel and the second access channel overlap at least partially.

### Detailed Description of the Embodiments

In Figure 1, reference numeral 1 relates to an ophthalmological device for surgical treatment of a cornea 20 of an eye 2 with a pulsed laser beam B.

As illustrated schematically in Figure 1, the ophthalmological device 1 comprises a laser source 11 for generating a pulsed laser beam B, a focusing optical module 12 for focusing the pulsed laser beam B in the cornea 20 onto a focus F, and a scanner system 13 for moving the focus F to target locations in the cornea 20.

In particular, the laser source 11 comprises a femtosecond laser for producing femtosecond laser pulses, which have pulse widths of typically 10 fs to 1000 fs (1 fs = 10⁻¹⁵s). The laser source 11 is arranged in a separate housing or in a housing shared with the focusing optical module 12.

The focusing optical module 12 is configured to focus the pulsed laser beam B and/or the laser pulses in the cornea 20 onto a focus F, i.e. for making the pulsed laser beam B converge to a focal point or spot in the cornea 20. The focusing optical module 12 comprises one or more optical lenses. By way of example, the focusing optical module 12 is installed in an application head 14, which can be placed onto the eye 2. The application head 14 or the focusing optical module 12, respectively, is preferably placed onto the eye 2 by way of an at least partly light-transparent contact body 15 or a fluid chamber and it is fastened to the eye 2 by means of a vacuum-controlled suction ring 16, for example, with the contact body 15 and the suction ring being 16 connected to the application head 14 in a fixed or removable manner. In an embodiment, the focusing optical module 12 comprises a focus adjustment device for setting the focal depth, for example one or more movable lenses, in the focusing optical module 12 or upstream of the focusing optical module 12, or a drive for moving the entire focusing optical module 12.

The scanner system 13 is configured to move the focus F to target locations in the cornea 20 by guiding and directing the pulsed laser beam B and thus the focus F to target locations in the cornea 20. The scanner system 13 comprises one or more scanner modules configured to guide and direct the pulsed laser beam B and thus the focus F in a x/y-work-plane which is normal to a z-axis, whereby the z-axis is aligned with or essentially parallel to a projection axis p of the focusing optical module 12, as illustrated schematically in Figure 1. Depending on the embodiment, the one or more scanner modules comprise one or more actuators configured to move the focusing optical module 12 such that the focus F is moved along a work line in the x/y-work-plane, and/or one or more deflection mirrors, each movable about one or two axes, configured to deflect the pulsed laser beam B and/or the laser pulses such that the focus F is moved along the work line in the x/y-work-plane. The scanner system 13 further comprises a z-modulator configured to move the focus F along the z-axis which is aligned with or essentially parallel to the projection axis p of the focusing optical module 12. For example, the z-modulator comprises a divergence modulator configured to dynamically change the divergence of the pulsed laser beam B. Various further and more specific embodiments of the scanner system 13 are described by the applicant in patent applications US 2019/0015250, US 2019/0015251, and US 2019/0015253.

The ophthalmological device 1 further comprises an electronic circuit 10 for controlling the laser source 11 and the scanner system 13. The electronic circuit 10 implements a programmable control device and comprises e.g. one or more processors 100 with program and data memory and programmed software modules for controlling the processors 100, and/or other programmable circuits or logic units such as ASICs (application specific integrated circuits).

The electronic circuit 10 is configured to control the scanner system 13 to move the focus F to cut inside the cornea 20 a lenticule L which has a posterior lenticule surface Lp and an anterior lenticule surface La, as illustrated in Figures 7-10. For example, the electronic circuit 10 is configured to control the scanner system 13 to move the focus F to cut the lenticule L inside the cornea 20 as described by the applicant in patent applications US 2019/0015250, US 2019/0015251, and US 2019/0015253.

The electronic circuit 10 is further configured to control the scanner system 13 to move the focus F to cut in the cornea 20 one or two opening incisions Co, Co1, Co2 in the exterior (anterior) surface A of the cornea 20, as illustrated in Figures 7-10. More specifically, the electronic circuit 10 is configured to control the scanner system 13 to move the focus F to cut in the exterior (anterior) surface A of the cornea 20 one common opening incision Co, as illustrated in Figures 7-9, or two separate opening incisions Co1, Co2, as illustrated in Figure 10.

Furthermore, the electronic circuit 10 is configured to control the scanner system 13 to move the focus F to cut in the cornea 20 access channels Ch1, Ch2 which connect the one or two opening incisions Co, Co1, Co2 to the posterior lenticule surface Lp and to the anterior lenticule surface La, respectively. More specifically, a first one of the access channels Ch1 connects the common opening incision Co or one of the two separate opening incisions Co1 to the posterior lenticule surface Lp. Moreover, a second one of the access channels Ch2 connects the common opening incision Co or one of the two separate opening incisions Co2 to the anterior lenticule surface La. The first access channel Ch1 is cut to approach tangentially the posterior lenticule surface Lp. The second access channel Ch2 is cut to approach tangentially the anterior lenticule surface La. The first access channel Ch1 enables a surgeon to access the posterior lenticule surface Lp by inserting a surgical tool, particularly a dissector, through the common opening incision Co or through one of the separate opening incisions Co1, and through the first access channel Ch1, making it possible for the surgeon to loosen and separate the posterior lenticule surface Lp from the neighbouring cornea tissue. The second access channels Ch2 enables the surgeon to access the anterior lenticule surface La by inserting the surgical tool, particularly the dissector, through the common opening incision Co or through one of the separate opening incisions Co2, and through the second access channel Ch2, making it possible for the surgeon to loosen and separate the anterior lenticule surface La from the neighbouring cornea tissue. In Figures 2-4, reference numeral (letter) k indicates the length of the access channel Ch1 from the opening incision Co to the posterior lenticule surface Lp. In Figures 2-4, reference numeral (letter) I indicates the length of the access channel Ch2 from the opening incision Co to the anterior lenticule surface La.

The electronic circuit 10 is configured to control the scanner system 13 to move the focus F to cut the access channels Ch1, Ch2 in the cornea 20 in such a fashion that the access channels Ch1, Ch2 overlap at least partially when viewed from a top view perspective onto the cornea 20, i.e. in a viewing direction along the z-axis, as illustrated in Figures 2-10.

In Figures 2-10, reference numeral (letter) E refers to a vertical plane which runs through the z-axis or the projection axis p, respectively. In Figures 7-10, the vertical plane E is assumed to coincide with the drawing plane.

As depicted in Figures 7-10, the access channels Ch1, Ch2 are each composed of two or more cut surfaces. The access channel Ch1 leading to the posterior lenticule surface Lp comprises a first cut surface which forms an entry channel q, extending from the opening incision Co, Co1 to a turning area Q with a change of direction (of the channel axis of access channel Ch1), and a second cut surface which forms a connecting channel, extending from the turning area Q to the posterior lenticule surface Lp. The access channel Ch2 leading to the anterior lenticule surface La comprises a first cut surface which forms an entry channel r, extending from the opening incision Co, Co2 to a turning area R with a change of direction R (of the channel axis of access channel Ch2), and one or more cut surfaces extending from the area with the change of direction R to the anterior lenticule surface La. As can be seen in Figures 2-10, the access channels Ch1, Ch2 and their cut surfaces run essentially normal to the vertical plane E.

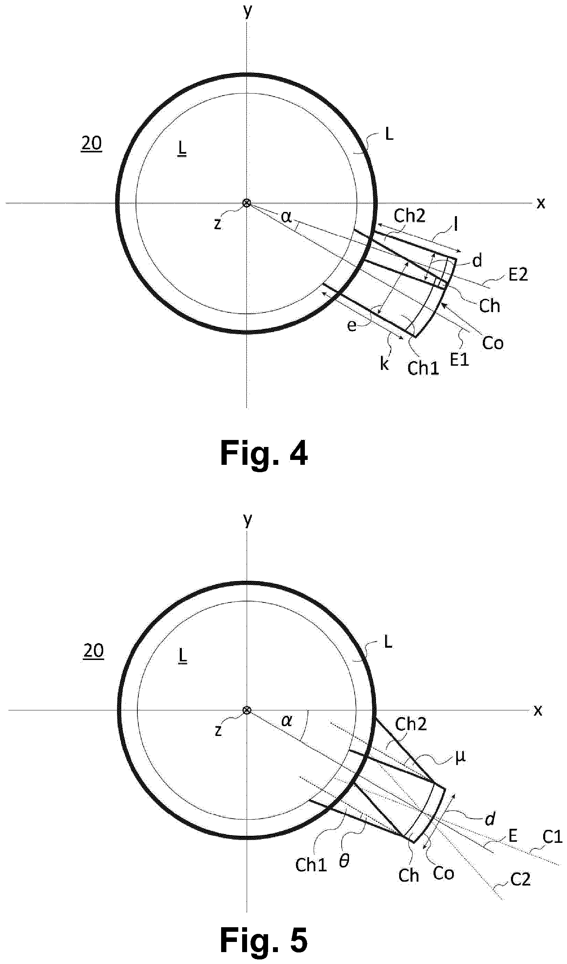

The electronic circuit 10 is further configured to control the scanner system 13 to move the focus F to cut the access channels Ch1, Ch2 in the cornea 20 in such a fashion that the access channels Ch1, Ch2 are produced with different geometric characteristics which enable a tactile distinction and differentiation of the access channels Ch1, Ch2 by the surgeon. For example, the access channels Ch1, Ch2 are produced with different channel widths d, e, i.e. with different widths d, e of their cut surfaces, as illustrated in Figures 2-4, and/or with different angular arrangements with regards to the vertical plane E, as illustrated in Figures 4-5, and/or with different location or placement, as illustrated in Figures 3-4. These different widths d, e, angular arrangements, and/or locations of the access channels Ch1, Ch2 can be detected in a tactile fashion by the surgeon inserting alternatingly a surgical tool, particularly a dissector, into the access channels Ch1, Ch2.

In the following paragraphs, different embodiments and/or configurations of the access channels Ch1, Ch2 are described with reference to Figures 2-6, showing top views of the access channels Ch1, Ch2, and to Figures 7-10, showing cross-sectional views of the access channels Ch1, Ch2. For the sake of clarity, it is pointed out here that any of the embodiments and/or configurations of the access channels Ch1, Ch2 illustrated in top view in Figures 2-6 can be combined with any of the embodiments and/or configurations of the access channels Ch1, Ch2 illustrated in cross-sectional in Figures 7-10. It is further particularly pointed out that, any of the embodiments and/or configurations of the access channels Ch1, Ch2 illustrated in top view in Figures 2-6 can be produced with one common opening incision Co, as illustrated in Figures 7-9, or, although not illustrated in Figures 2-6, with two separate opening incisions Co1, Co2, as illustrated in Figure 10. Finally, it is pointed out that the electronic circuit 10 is configured to control the scanner system 13 to move the focus F to cut the access channels Ch1, Ch2 in the cornea 20 to produce one or more of these embodiments and/or configurations and combinations thereof, for example, as selected or selectable by an operator.

In the embodiment illustrated in Figure 2, the access channels Ch1 and Ch2 have a common symmetry plane E=E1=E2 which coincides with the vertical plane E. As further illustrated, the access channels have Ch1, Ch2 a different channel width d and e, respectively, making possible for the surgeon a tactile distinction of the two access channels Ch1, Ch2. The channel width d, e is defined by the widths of the cut surfaces forming the access channels have Ch1, Ch2. As can be seen in Figures 2-4, the channel width d, e is defined by the extension of the cut surfaces forming the access channels Ch1, Ch2 with respect to the symmetry plane E1, E2 of the respective access channel Ch1, Ch2.

In the embodiment illustrated in Figure 3, the access channels Ch1 and Ch2 have each their own separate symmetry plane E1, E2 which run parallel to the vertical plane E and thus parallel to each other. As in the embodiment of Figure 2, the access channels Ch1, Ch2 have a different channel width d, e and are further distinguishable in a tactile fashion for the surgeon by their location, as they have separate symmetry planes E1, E2 and are thus offset or shifted with respect to each other.

In the embodiment illustrated in Figure 4, the access channels Ch1 and Ch2 have each their own separate symmetry plane E1, E2 which run at an angle α to each other (from the top view perspective). As in the embodiments of Figures 2 and 3, the access channels Ch1, Ch2 have a different channel width d, e and are further distinguishable in a tactile fashion for the surgeon by their angular arrangement with respect to each other.

In the embodiment illustrated in Figure 5, the access channels Ch1 and Ch2 have each their own separate central plane C1, C2 which run parallel to the z-axis or the projection axis p, respectively, with a different angle Θ or µ, respectively, to the vertical plane E and thus forming and enclosing an angle Θ+µ between each other (from the top view perspective). Unlike in the embodiments of Figure 2 and 3, the access channels Ch1, Ch2 have the same channel width d, but are distinguishable in a tactile fashion for the surgeon by their angular arrangement with respect to each other.

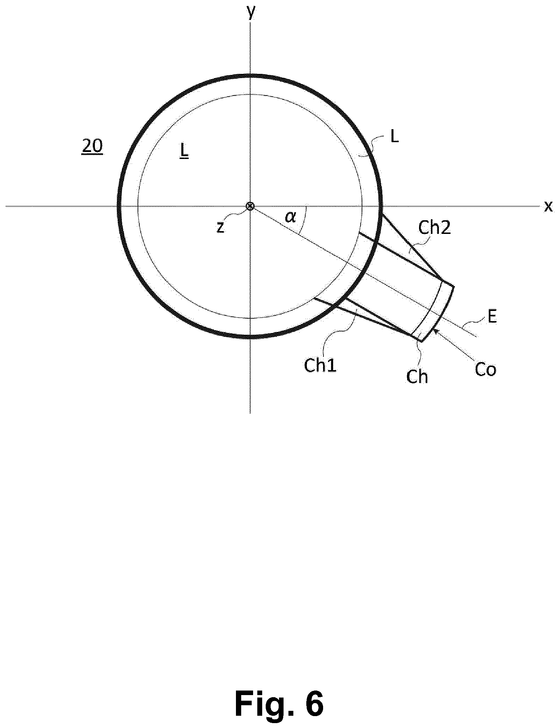

In the embodiment illustrated in Figure 6, the access channels Ch1 and Ch2 have a trapezoid shape, from the top view perspective. The access channels Ch1 and Ch2 are arranged at an angle to each other (from the top view perspective) and are thus distinguishable in a tactile fashion for the surgeon by their angular arrangement with respect to each other.

Figures 7-10 show in schematic cross-sectional view, where the drawing plane is assumed to coincide with the vertical plane E, different arrangements of the cut surfaces forming the access channels Ch1, Ch2.

In the embodiment illustrated in Figure 7, the access channels Ch1 and Ch2 share a common entry channel r, extending from the common opening incision Co to the turning area R. The common entry channel r is produced by a first cut surface which is cut from the common opening incision Co into the cornea tissue at an entry angle ϕ with respect to the anterior surface A of the cornea 20. At the turning area R, the access channel Ch2 changes direction to a connection channel with length I, from the turning area R to the anterior lenticule surface La, formed by second cut surface. For the access channel Ch1 to the posterior lenticule surface Lp, the entry channel r continues as entry channel q from the turning area R to the turning area Q, formed by the continued first cut surface. At the turning area Q, the access channel Ch1 changes direction to a connection channel with length k, from the turning area Q to the posterior lenticule surface Lp, formed by third cut surface.

In the embodiment illustrated in Figure 8, the access channels Ch1 and Ch2 have separate entry channels r and q, extending from the common opening incision Co to the turning area R or the turning area Q, respectively. The entry channel q for access channel Ch1 is produced by a first cut surface which is cut from the common opening incision Co into the cornea tissue at an entry angle ϕ with respect to the anterior surface A of the cornea 20. At the turning area Q, the access channel Ch1 changes direction to a connection channel with length k, from the turning area Q to the posterior lenticule surface Lp, formed by a second cut surface. The entry channel r for the access channel Ch2 to the anterior lenticule surface La is produced by a third cut surface which is cut from the common opening incision Co into the cornea tissue at an entry angle with respect to the entry channel q for the access channel Ch1. At the turning area R, the access channel Ch2 changes direction to a connection channel with length I, from the turning area R to the anterior lenticule surface La, formed by a fourth cut surface.

In the embodiment illustrated in Figure 9, the access channels Ch1 and Ch2 share a common entry channel r, extending with length s from the common opening incision Co to the turning area R. The common entry channel r is produced by a first cut surface which is cut from the common opening incision Co into the cornea tissue at an entry angle ϕ with respect to the anterior surface A of the cornea 20. At the turning area R, the access channel Ch2 changes direction to a two-part connection channel formed by a second and a third cut surface, from the turning area R to the anterior lenticule surface La. For the access channel Ch1 to the posterior lenticule surface Lp, the entry channel r continues as entry channel q from the turning area R to the turning area Q, formed by the continued first cut surface. At the turning area Q, the access channel Ch1 changes direction to a connection channel with length k, from the turning area Q to the posterior lenticule surface Lp, formed by a fourth cut surface.

In the embodiment illustrated in Figure 10, the access channels Ch1 and Ch2 have separate entry channels r and q, extending from separate opening incisions Co1 or Co2, respectively, to the turning area R or the turning area Q, respectively. The entry channel q for access channel Ch1 is produced by a first cut surface which is cut from the opening incision Co1 into the cornea tissue at an entry angle ϕ with respect to the anterior surface A of the cornea 20. At the turning area Q, the access channel Ch1 changes direction to a connection channel with length k, from the turning area Q to the posterior lenticule surface Lp, formed by second cut surface. The entry channel r for the access channel Ch2 to the anterior lenticule surface La is produced by a third cut surface which is cut from the opening incision Co2 into the cornea tissue at an entry angle ω with respect to the anterior surface A of the cornea 20. At the turning area R, the access channel Ch2 changes direction to a connection channel with length I, from the turning area R to the anterior lenticule surface La, formed by a fourth cut surface.

## Claims

1. An ophthalmological device (1) for surgical treatment of a cornea (20) of an eye (2), the ophthalmological device (1) comprising:
a laser source (11) configured to generate a pulsed laser beam (B);
a focusing optical module (12) configured to make the pulsed laser beam (B) converge onto a focus (F) in the cornea (20);
a scanner system (13) configured to move the focus to target locations in the cornea (20); and
an electronic circuit (10) configured to control the scanner system (13) to move the focus (F) to cut inside the cornea (20) a lenticule (L), the lenticule (L) having a posterior lenticule surface (Lp) and an anterior lenticule surface (La),
wherein the electronic circuit (10) is further configured to control the scanner system (13) to move the focus (F) to cut in the cornea (20) at least one opening incision (Co) in an exterior surface (A) of the cornea (20), a first access channel (Ch1), and a second access channel (Ch2), the first access channel (Ch1) connecting the at least one opening incision (Co) to the posterior lenticule surface (Lp) to provide access for a surgical tool through the at least one opening incision (Co) to the posterior lenticule surface (Lp), and the second access channel (Ch2) connecting the at least one opening incision (Co) to the anterior lenticule surface (La) to provide access for the surgical tool through the at least one opening incision (Co) to the anterior lenticule surface (La), whereby the first access channel (Ch1) and the second access channel (Ch2) overlap at least partially from a top view perspective onto the cornea (20).

2. The ophthalmological device (1) of claim 1, wherein the electronic circuit (10) is configured to control the scanner system (13) to move the focus (F) to cut one common opening incision (Co) in the exterior surface of the cornea (20) for the first access channel (Ch1) and the second access channel (Ch2), to provide access for the surgical tool through the common opening incision (Co) and the first access channel (Ch1) to the posterior lenticule surface (Lp), and through the common opening incision (Co) and the second access channel (Ch2) to the anterior lenticule surface (La).

3. The ophthalmological device (1) of claim 2, wherein the electronic circuit (10) is further configured to control the scanner system (13) to move the focus (F) to cut the first access channel (Ch1) and the second access channel (Ch2) with a partially common access channel to provide access for the surgical tool through the common opening incision (Co), the common access channel and the first access channel (Ch1) to the posterior lenticule surface (Lp), and through the common opening incision (Co), the common access channel and the second access channel (Ch2) to the anterior lenticule surface (La).

4. The ophthalmological device (1) of claim 1, wherein the electronic circuit (10) is configured to control the scanner system (13) to move the focus (F) to cut in the cornea (20) a first opening incision (Co1) in the exterior surface of the cornea (20) for the first access channel (Ch1) to provide access for the surgical tool through the first opening incision (Co1) and the first access channel (Ch1) to the posterior lenticule surface (Lp), and a separate second opening incision (Co2) in the exterior surface (A) of the cornea (20) for the second access channel (Ch2) to provide access for the surgical tool through the second opening incision (Co2) and the second access channel (Ch2) to the anterior lenticule surface (La).

5. The ophthalmological device (1) of one of claims 1 to 4, wherein the electronic circuit (10) is configured to control the scanner system (13) to move the focus (F) to cut the first access channel (Ch1) in the cornea (20) with a first width (d), and to cut the second access channel (Ch2) in the cornea (20) with a second width (e) different from the first width (d).

6. The ophthalmological device (1) of one of claims 1 to 5, wherein the electronic circuit (10) is configured to control the scanner system (13) to move the focus (F) to cut the first access channel (Ch1) in the cornea (20) with a first symmetry plane (E1), and to cut the second access channel (Ch2) in the cornea (20) with a second symmetry plane (E2), whereby the first symmetry plane (E1) and the second symmetry plane (E2) run parallel to each other.

7. The ophthalmological device (1) of one of claims 1 to 6, wherein the electronic circuit (10) is configured to control the scanner system (13) to move the focus (F) to cut the first access channel (Ch1) in the cornea (20) with a first symmetry plane (E1), and to cut the second access channel (Ch2) in the cornea (20) with a second symmetry plane (E2), whereby the first symmetry plane (E1) and the second symmetry plane (E2) run at an angle to each other.

8. The ophthalmological device (1) of one of claims 1 to 7, wherein the electronic circuit (10) is configured to control the scanner system (13) to move the focus (F) to cut the first access channel (Ch1) and the second access channel (Ch2) with a trapezoid shaped outline from the top view perspective onto the cornea (20).

9. The ophthalmological device (1) of one of claims 1 to 8, wherein the electronic circuit (10) is configured to control the scanner system (13) to move the focus (F) to cut at least one of the first access channel (Ch1) or the second access channel (Ch2) in the cornea (20) from with an increasing width from the opening incision (Co) in the exterior surface of the cornea (20) towards the posterior lenticule surface (Lp) or the anterior lenticule surface (La), respectively.

10. The ophthalmological device (1) of one of claims 1 to 9, wherein the electronic circuit (10) is configured to control the scanner system (13) to move the focus (F) to cut the first access channel (Ch1) along a first channel axis (q) having at least one change of direction (Q) from the opening incision (Co) in the exterior surface (A) of the cornea (20) to the posterior lenticule surface (Lp), and to cut the second access channel (Ch2) along a second channel axis (r) having at least one change of direction (R) from the opening incision (Co) in the exterior surface (A) of the cornea (20) to the anterior lenticule surface (La).

11. The ophthalmological device (1) of one of claims 1 to 10, wherein the electronic circuit (10) is configured to control the scanner system (13) to move the focus (F) to cut the first access channel (Ch1) to approach tangentially the posterior lenticule surface (Lp), and to cut the second access channel (Ch2) to approach tangentially the anterior lenticule surface (La).

12. The ophthalmological device (1) of one of claims 1 to 11, wherein the electronic circuit (10) is configured to control the scanner system (13) to move the focus (F) to cut the first access channel (Ch1) with a first entry channel, from the at least one opening incision (Co) in the exterior surface (A) of the cornea (20) to a first turning area (Q) inside the cornea (20), and a first connecting channel from the first turning area (Q) to the posterior lenticule surface (Lp), and to cut the second access channel (Ch2) with a second entry channel from the at least one opening incision (Co) in the exterior surface (A) of the cornea (20) to a second turning area (R) inside the cornea (20), and a second connecting channel from the second turning area (R) to the anterior lenticule surface (La).

13. The ophthalmological device (1) of claim 12, wherein the electronic circuit (10) is configured to control the scanner system (13) to move the focus (F) to cut the first entry channel and the second entry channel with a common channel portion from the at least one opening incision (Co) in the exterior surface of the cornea (20) to a forking area inside the cornea (20).

14. A computer program product comprising a non-transitory computer-readable medium having stored thereon computer program code for controlling a processor of an ophthalmological device (1) which comprises a laser source configured to generate a pulsed laser beam, a focusing optical module configured to make the pulsed laser beam converge onto a focus (F) in the cornea (20), and a scanner system (13) configured to move the focus (F) to target locations in the cornea (20), whereby the computer program code is configured to control the processor such that the processor:
directs the scanner system (13) to move the focus (F) to cut inside the cornea (20) a lenticule, the lenticule having a posterior lenticule surface (Lp) and an anterior lenticule surface (La), and to move the focus (F) according to cut in the cornea (20) at least one opening incision (Co), a first access channel (Ch1), and a second access channel (Ch2), the first access channel (Ch1) connecting the at least one opening incision (Co) to the posterior lenticule surface (Lp) to provide access for a surgical tool through the at least one opening incision (Co) to the posterior lenticule surface (Lp), and the second access channel (Ch2) connecting the at least one opening incision (Co) to the anterior lenticule surface (La) to provide access for the surgical tool through the at least one opening incision (Co) to the anterior lenticule surface (La), whereby the first access channel (Ch1) and the second access channel (Ch2) overlap at least partially from a top view perspective onto the cornea (20).

## Patentansprüche

1. Ophthalmologische Vorrichtung (1) zur chirurgischen Behandlung einer Hornhaut (20) eines Auges (2), wobei die ophthalmologische Vorrichtung (1) aufweist:
eine Laserquelle (11), die ausgebildet ist, um einen gepulsten Laserstrahl (B) zu generieren;
ein Fokussieroptikmodul (12), das ausgebildet ist, um den gepulsten Laserstrahl (B) zum Konvergieren auf einem Brennpunkt (F) in der Hornhaut (20) zu bringen;
ein Scannersystem (13), das ausgebildet ist, um den Brennpunkt auf Zielpositionen in der Hornhaut (20) zu bewegen; und
eine elektronische Schaltung (10), die ausgebildet ist, um das Scannersystem (13) zu steuern, um den Brennpunkt (F) zu bewegen, um innerhalb der Hornhaut (20) ein Lentikel (L) zu schneiden, wobei das Lentikel eine posteriore Lentikeloberfläche (Lp) und eine anteriore Lentikeloberfläche (La) aufweist,
wobei die elektronische Schaltung (10) des Weiteren ausgebildet ist, um das Scannersystem (13) zu steuern, um den Brennpunkt (F) zu bewegen, um in die Hornhaut (20) mindestens eine öffnende Inzision (Co) in eine Außenoberfläche (A) der Hornhaut (20), einen ersten Zugangskanal (Ch1) und einen zweiten Zugangskanal (Ch2) zu schneiden, wobei der erste Zugangskanal (Ch1) die mindestens eine öffnende Inzision (Co) mit der posterioren Lentikeloberfläche (Lp) verbindet, um Zugang für ein chirurgisches Werkzeug durch die mindestens eine öffnende Inzision (Co) hindurch zu der posterioren Lentikeloberfläche (Lp) bereitzustellen, und der zweite Zugangskanal (Ch2) die mindestens eine öffnende Inzision (Co) mit der anterioren Lentikeloberfläche (La) verbindet, um Zugang für das chirurgische Werkzeug durch die mindestens eine öffnende Inzision (Co) hindurch zu der anterioren Lentikeloberfläche (La) bereitzustellen, wobei der erste Zugangskanal (Ch1) und der zweite Zugangskanal (Ch2) sich in der Draufsichtperspektive auf die Hornhaut (20) mindestens teilweise überlappen.

2. Ophthalmologische Vorrichtung (1) nach Anspruch 1, wobei die elektronische Schaltung (10) ausgebildet ist, um das Scannersystem (13) zu steuern, um den Brennpunkt (F) zu bewegen, um eine gemeinsame öffnende Inzision (Co) in die Außenoberfläche der Hornhaut (20) für den ersten Zugangskanal (Ch1) und den zweiten Zugangskanal (Ch2) zu schneiden, um Zugang für das chirurgische Werkzeug durch die gemeinsame öffnende Inzision (Co) und den ersten Zugangskanal (Ch1) zu der posterioren Lentikeloberfläche (Lp) und durch die gemeinsame öffnende Inzision (Co) und den zweiten Zugangskanal (Ch2) zu der anterioren Lentikeloberfläche (La) bereitzustellen.

3. Ophthalmologische Vorrichtung (1) nach Anspruch 2, wobei die elektronische Schaltung (10) des Weiteren ausgebildet ist, um das Scannersystem (13) zu steuern, um den Brennpunkt (F) zu bewegen, um den ersten Zugangskanal (Ch1) und den zweiten Zugangskanal mit einem teilweise gemeinsamen Zugangskanal zu schneiden, um Zugang bereitzustellen für das chirurgische Werkzeug durch die gemeinsame öffnende Inzision (Co), den gemeinsamen Zugangskanal und den ersten Zugangskanal (Ch1) zu der posterioren Lentikeloberfläche (Lp) und durch die gemeinsame öffnende Inzision (Co), den gemeinsamen Zugangskanal und den zweiten Zugangskanal (Ch2) zu der anterioren Lentikeloberfläche (La).

4. Ophthalmologische Vorrichtung (1) nach Anspruch 1, wobei die elektronische Schaltung (10) ausgebildet ist, um das Scannersystem (13) zu steuern, um den Brennpunkt (F) zu bewegen, um in die Hornhaut (20) eine erste öffnende Inzision (Co1) in der Außenoberfläche der Hornhaut (20) für den ersten Zugangskanal (Ch1) zu schneiden, um Zugang für das chirurgische Werkzeug durch die erste öffnende Inzision (Co1) und den ersten Zugangskanal (Ch1) zu der posterioren Lentikeloberfläche (Lp) bereitzustellen, und eine separate zweite öffnende Inzision (Co2) in die Außenoberfläche (A) der Hornhaut (20) für den zweiten Zugangskanal (Ch2) zu schneiden, um Zugang für das chirurgische Werkzeug durch die zweite öffnende Inzision (Co2) und den zweiten Zugangskanal (Ch2) zu der anterioren Lentikeloberfläche (La) bereitzustellen.

5. Ophthalmologische Vorrichtung (1) nach einem der Ansprüche 1 bis 4, wobei die elektronische Schaltung (10) ausgebildet ist, um das Scannersystem (13) zu steuern, um den Brennpunkt (F) zu bewegen, um den ersten Zugangskanal (Ch1) in der Hornhaut (20) mit einer ersten Breite (d) zu schneiden, und um den zweiten Zugangskanal (Ch2) in der Hornhaut (20) mit einer zweiten Breite (e) zu schneiden, die sich von der ersten Breite (d) unterscheidet.

6. Ophthalmologische Vorrichtung (1) nach einem der Ansprüche 1 bis 5, wobei die elektronische Schaltung (10) ausgebildet ist, um das Scannersystem (13) zu steuern, um den Brennpunkt (F) zu bewegen, um den ersten Zugangskanal (Ch1) in der Hornhaut (20) mit einer ersten Symmetrieebene (E1) zu schneiden, und um den zweiten Zugangskanal (Ch2) in der Hornhaut (20) mit einer zweiten Symmetrieebene (E2) zu schneiden, wobei die erste Symmetrieebene (E1) und die zweite Symmetrieebene (E2) parallel zueinander verlaufen.

7. Ophthalmologische Vorrichtung (1) nach einem der Ansprüche 1 bis 6, wobei die elektronische Schaltung (10) ausgebildet ist, um das Scannersystem (13) zu steuern, um den Brennpunkt (F) zu bewegen, um den ersten Zugangskanal (Ch1) in der Hornhaut (20) mit einer ersten Symmetrieebene (E1) zu schneiden, und um den zweiten Zugangskanal (Ch2) in der Hornhaut (20) mit einer zweiten Symmetrieebene (E2) zu schneiden, wobei die erste Symmetrieebene (E1) und die zweite Symmetrieebene (E2) in einem Winkel zueinander verlaufen.

8. Ophthalmologische Vorrichtung (1) nach einem der Ansprüche 1 bis 7, wobei die elektronische Schaltung (10) ausgebildet ist, um das Scannersystem (13) zu steuern, um den Brennpunkt (F) zu bewegen, um den ersten Zugangskanal (Ch1) und den zweiten Zugangskanal (Ch2) mit einem trapezförmigen Umriss aus der Draufsichtperspektive auf die Hornhaut (20) zu schneiden.

9. Ophthalmologische Vorrichtung (1) nach einem der Ansprüche 1 bis 8, wobei die elektronische Schaltung (10) ausgebildet ist, um das Scannersystem (13) zu steuern, um den Brennpunkt (F) zu bewegen, um mindestens einen von dem ersten Zugangskanal (Ch1) oder dem zweiten Zugangskanal (Ch2) in der Hornhaut (20) mit einer zunehmenden Breite von der öffnenden Inzision (Co) in der Außenoberfläche der Hornhaut (20) in Richtung der posterioren Lentikeloberfläche (Lp) beziehungsweise der anterioren Lentikeloberfläche (La) zu schneiden.

10. Ophthalmologische Vorrichtung (1) nach einem der Ansprüche 1 bis 9, wobei die elektronische Schaltung (10) ausgebildet ist, um das Scannersystem (13) zu steuern, um den Brennpunkt (F) zu bewegen, um den ersten Zugangskanal (Ch1) entlang einer ersten Kanalachse (q) mit mindestens einer Änderung der Richtung (Q) von der öffnenden Inzision (Co) in der Außenoberfläche (A) der Hornhaut (20) zu der posterioren Lentikeloberfläche (Lp) zu schneiden, und um den zweiten Zugangskanal (Ch2) entlang einer zweiten Kanalachse (r) mit mindestens einer Richtungsänderung (R) von der öffnenden Inzision (Co) in der Außenoberfläche (A) der Hornhaut (20) zu der anterioren Lentikeloberfläche (La) zu schneiden.

11. Ophthalmologische Vorrichtung (1) nach einem der Ansprüche 1 bis 10, wobei die elektronische Schaltung (10) ausgebildet ist, um das Scannersystem (13) zu steuern, um den Brennpunkt (F) zu bewegen, um den ersten Zugangskanal (Ch1) zu schneiden, um sich der posterioren Lentikeloberfläche (Lp) tangential zu nähern, und um den zweiten Zugangskanal (Ch2) zu schneiden, um sich der anterioren Lentikeloberfläche (La) tangential zu nähern.

12. Ophthalmologische Vorrichtung (1) nach einem der Ansprüche 1 bis 11, wobei die elektronische Schaltung (10) ausgebildet ist, um das Scannersystem (13) zu steuern, um den Brennpunkt (F) zu bewegen, um den ersten Zugangskanal (Ch1) mit einem ersten Eintrittskanal von der mindestens einen öffnenden Inzision (Co) in der Außenoberfläche (A) der Hornhaut (20) zu einem ersten Wendebereich (Q) innerhalb der Hornhaut (20) und einen ersten Verbindungskanal von dem ersten Wendebereich (Q) zu der posterioren Lentikeloberfläche zu schneiden, und um den zweiten Zugangskanal (Ch2) mit einem zweiten Eintrittskanal von der mindestens einen öffnenden Inzision (Co) in der Außenoberfläche (A) der Hornhaut (20) zu einem zweiten Wendebereich (R) innerhalb der Hornhaut (20) und einen zweiten Verbindungskanal von dem zweiten Wendebereich (R) zu der anterioren Lentikeloberfläche (La) zu schneiden.

13. Ophthalmologische Vorrichtung (1) nach Anspruch 12, wobei die elektronische Schaltung (10) ausgebildet ist, um das Scannersystem (13) zu steuern, um den Brennpunkt (F) zu bewegen, um den ersten Eintrittskanal und den zweiten Eintrittskanal mit einem gemeinsamen Kanalabschnitt von der mindestens einen öffnenden Inzision (Co) in der Außenoberfläche der Hornhaut (20) zu einem Gabelungsbereich innerhalb der Hornhaut (20) zu schneiden.

14. Computerprogrammprodukt, aufweisend ein nichtflüchtiges computerlesbares Medium, auf dem Computerprogrammcode zum Steuern eines Prozessors einer ophthalmologischen Vorrichtung (1) gespeichert ist, die eine Laserquelle, die ausgebildet ist, um einen gepulsten Laserstrahl zu generieren, ein Fokussieroptikmodul, das ausgebildet ist, um den gepulsten Laserstrahl zum Konvergieren auf einen Brennpunkt (F) in der Hornhaut (20) zu bringen, und ein Scannersystem (13) aufweist, das ausgebildet ist, um den Brennpunkt (F) auf Zielpositionen in der Hornhaut (20) zu bewegen, wobei der Computerprogrammcode ausgebildet ist, um den Prozessor zu steuern, so dass der Prozessor folgendes ausführt:
Lenken des Scannersystems (13), um den Brennpunkt (F) zu bewegen, um in der Hornhaut (20) ein Lentikel zu schneiden, wobei das Lentikel eine posteriore Lentikeloberfläche (Lp) und eine anteriore Lentikeloberfläche (La) hat, und um den Brennpunkt (F) dementsprechend zu bewegen, um in die Hornhaut (20) mindestens eine öffnende Inzision (Co), einen ersten Zugangskanal (Ch1) und einen zweiten Zugangskanal (Ch2) zu schneiden, wobei der erste Zugangskanal (Ch1) die mindestens eine öffnende Inzision (Co) mit der posterioren Lentikeloberfläche (Lp) verbindet, um Zugang für ein chirurgisches Werkzeug durch die mindestens eine öffnende Inzision (Co) hindurch zu der posterioren Lentikeloberfläche (Lp) bereitzustellen, und der zweite Zugangskanal (Ch2) die mindestens eine öffnende Inzision (Co) mit der anterioren Lentikeloberfläche (La) verbindet, um Zugang für das chirurgische Werkzeug durch die mindestens eine öffnende Inzision (Co) hindurch zu der anterioren Lentikeloberfläche (La) bereitzustellen, wobei der erste Zugangskanal (Ch1) und der zweite Zugangskanal (Ch2) einander in der Draufsichtperspektive auf die Hornhaut (20) mindestens teilweise überlappen.

## Revendications

1. Dispositif ophtalmologique (1) pour le traitement chirurgical d'une cornée (20) d'un œil (2), le dispositif ophtalmologique (1) comprenant :
une source laser (11) configurée pour générer un faisceau laser pulsé (B) ;
un module optique de focalisation (12) configuré pour faire converger le faisceau laser pulsé (B) vers un foyer (F) dans la cornée (20) ;
un système de balayage (13) configuré pour déplacer le foyer vers des emplacements cibles dans la cornée (20) ; et
un circuit électronique (10) configuré pour commander le système de balayage (13) afin de déplacer le foyer (F) pour couper à l'intérieur de la cornée (20) un lenticule (L), le lenticule (L) ayant une surface de lenticule postérieure (Lp) et une surface de lenticule antérieure (La),
le circuit électronique (10) étant en outre configuré pour commander le système de balayage (13) afin de déplacer le foyer (F) pour couper dans la cornée (20) au moins une incision d'ouverture (Co) dans une surface extérieure (A) de la cornée (20), un premier canal d'accès (Ch1) et un second canal d'accès (Ch2), le premier canal d'accès (Ch1) reliant l'au moins une incision d'ouverture (Co) à la surface de lenticule postérieure (Lp) pour permettre l'accès d'un outil chirurgical à la surface de lenticule postérieure (Lp) à travers l'au moins une incision d'ouverture (Co), et le second canal d'accès (Ch2) reliant l'au moins une incision d'ouverture (Co) à la surface de lenticule antérieure (La) pour permettre l'accès de l'outil chirurgical à la surface de lenticule antérieure (La), à travers l'au moins une incision d'ouverture (Co), de sorte que le premier canal d'accès (Ch1) et le second canal d'accès (Ch2) se chevauchent au moins partiellement en vue de dessus sur la cornée (20).

2. Dispositif ophtalmologique (1) selon la revendication 1, le circuit électronique (10) étant configuré pour commander le système de balayage (13) afin de déplacer le foyer (F) pour couper une incision d'ouverture commune (Co) dans la surface extérieure de la cornée (20) pour le premier canal d'accès (Ch1) et le second canal d'accès (Ch2), afin de permettre l'accès de l'outil chirurgical à la surface de lenticule postérieure (Lp), à travers l'incision d'ouverture commune (Co) et le premier canal d'accès (Ch1), et, à la surface de lenticule antérieure (La), à travers l'incision d'ouverture commune (Co) et le second canal d'accès (Ch2),.

3. Dispositif ophtalmologique (1) selon la revendication 2, le circuit électronique (10) étant en outre configuré pour commander le système de balayage (13) afin de déplacer le foyer (F) pour couper le premier canal d'accès (Ch1) et le second canal d'accès (Ch2) avec un canal d'accès partiellement commun afin de permettre l'accès de l'outil chirurgical à la surface de lenticule postérieure (Lp), à travers l'incision d'ouverture commune (Co), le canal d'accès commun et le premier canal d'accès (Ch1), et à la surface de lenticule antérieure (La), à travers l'incision d'ouverture commune (Co), le canal d'accès commun et le second canal d'accès (Ch2).

4. Dispositif ophtalmologique (1) selon la revendication 1, le circuit électronique (10) étant configuré pour commander le système de balayage (13) afin de déplacer le foyer (F) pour couper dans la cornée (20) une première incision d'ouverture (Co1) dans la surface extérieure de la cornée (20) pour le premier canal d'accès (Ch1) afin de permettre l'accès de l'outil chirurgical à la surface de lenticule postérieure (Lp), à travers la première incision d'ouverture (Co1) et le premier canal d'accès (Ch1), et une seconde incision d'ouverture (Co2) séparée dans la surface extérieure (A) de la cornée (20) pour le second canal d'accès (Ch2) afin de permettre l'accès de l'outil chirurgical à la surface de lenticule antérieure (La), à travers la seconde incision d'ouverture (Co2) et le second canal d'accès (Ch2).

5. Dispositif ophtalmologique (1) selon l'une des revendications 1 à 4, le circuit électronique (10) étant configuré pour commander le système de balayage (13) afin de déplacer le foyer (F) pour couper le premier canal d'accès (Ch1) dans la cornée (20) avec une première largeur (d), et pour couper le second canal d'accès (Ch2) dans la cornée (20) avec une seconde largeur (e) différente de la première largeur (d).

6. Dispositif ophtalmologique (1) selon l'une des revendications 1 à 5, le circuit électronique (10) étant configuré pour commander le système de balayage (13) afin de déplacer le foyer (F) pour couper le premier canal d'accès (Ch1) dans la cornée (20) avec un premier plan de symétrie (E1), et pour couper le second canal d'accès (Ch2) dans la cornée (20) avec un second plan de symétrie (E2), le premier plan de symétrie (E1) et le second plan de symétrie (E2) étant parallèles l'un à l'autre.

7. Dispositif ophtalmologique (1) selon l'une des revendications 1 à 6, le circuit électronique (10) étant configuré pour commander le système de balayage (13) afin de déplacer le foyer (F) pour couper le premier canal d'accès (Ch1) dans la cornée (20) avec un premier plan de symétrie (E1), et pour couper le second canal d'accès (Ch2) dans la cornée (20) avec un second plan de symétrie (E2), le premier plan de symétrie (E1) et le second plan de symétrie (E2) formant un angle l'un par rapport à l'autre.

8. Dispositif ophtalmologique (1) selon l'une des revendications 1 à 7, le circuit électronique (10) étant configuré pour commander le système de balayage (13) afin de déplacer le foyer (F) pour couper le premier canal d'accès (Ch1) et le second canal d'accès (Ch2) avec un contour en forme de trapèze depuis la vue de dessus sur la cornée (20).

9. Dispositif ophtalmologique (1) selon l'une des revendications 1 à 8, le circuit électronique (10) étant configuré pour commander le système de balayage (13) afin de déplacer le foyer (F) pour couper au moins l'un du premier canal d'accès (Ch1) ou du second canal d'accès (Ch2) dans la cornée (20) avec une largeur croissante à partir de l'incision d'ouverture (Co) dans la surface extérieure de la cornée (20) vers la surface de lenticule postérieure (Lp) ou la surface de lenticule antérieure (La), respectivement.

10. Dispositif ophtalmologique (1) selon l'une des revendications 1 à 9, le circuit électronique (10) étant configuré pour commander le système de balayage (13) afin de déplacer le foyer (F) pour couper le premier canal d'accès (Ch1) le long d'un premier axe de canal (q) ayant au moins un changement de direction (Q) de l'incision d'ouverture (Co) dans la surface extérieure (A) de la cornée (20) vers la surface de lenticule postérieure (Lp), et pour couper le second canal d'accès (Ch2) le long d'un second axe de canal (r) ayant au moins un changement de direction (R) de l'incision d'ouverture (Co) dans la surface extérieure (A) de la cornée (20) vers la surface de lenticule antérieure (La).

11. Dispositif ophtalmologique (1) selon l'une des revendications 1 à 10, le circuit électronique (10) étant configuré pour commander le système de balayage (13) afin de déplacer le foyer (F) pour couper le premier canal d'accès (Ch1) afin d'approcher tangentiellement la surface de lenticule postérieure (Lp), et pour couper le second canal d'accès (Ch2) afin d'approcher tangentiellement la surface de lenticule antérieure (La).

12. Dispositif ophtalmologique (1) selon l'une des revendications 1 à 11, le circuit électronique (10) étant configuré pour commander le système de balayage (13) afin de déplacer le foyer (F) pour couper le premier canal d'accès (Ch1) avec un premier canal d'entrée, depuis l'au moins une incision d'ouverture (Co) dans la surface extérieure (A) de la cornée (20) jusqu'à une première zone de rotation (Q) à l'intérieur de la cornée (20), et un premier canal de connexion depuis la première zone de rotation (Q) jusqu'à la surface de lenticule postérieure (Lp), et de couper le second canal d'accès (Ch2) avec un second canal d'entrée depuis l'au moins une incision d'ouverture (Co) dans la surface extérieure (A) de la cornée (20) jusqu'à une seconde zone de rotation (R) à l'intérieur de la cornée (20), et un second canal de connexion depuis la seconde zone de rotation (R) jusqu'à la surface de lenticule antérieure (La).

13. Dispositif ophtalmologique (1) selon la revendication 12, le circuit électronique (10) étant configuré pour commander le système de balayage (13) afin de déplacer le foyer (F) pour couper le premier canal d'entrée et le second canal d'entrée avec une portion de canal commune depuis l'au moins une incision d'ouverture (Co) dans la surface extérieure de la cornée (20) jusqu'à une zone de bifurcation à l'intérieur de la cornée (20).

14. Produit de programme informatique comprenant un support lisible par ordinateur non transitoire sur lequel est stocké un code de programme informatique pour commander un processeur d'un dispositif ophtalmologique (1) qui comprend une source laser configurée pour générer un faisceau laser pulsé, un module optique de focalisation configuré pour faire converger le faisceau laser pulsé vers un foyer (F) dans la cornée (20), et un système de balayage (13) configuré pour déplacer le foyer (F) vers des emplacements cibles dans la cornée (20), le code de programme informatique étant configuré pour commander le processeur de manière à ce que le processeur :
ordonne au système de balayage (13) de déplacer le foyer (F) pour couper à l'intérieur de la cornée (20) un lenticule, le lenticule ayant une surface de lenticule postérieure (Lp) et une surface de lenticule antérieure (La), et de déplacer le foyer (F) afin de couper dans la cornée (20) au moins une incision d'ouverture (Co), un premier canal d'accès (Ch1) et un second canal d'accès (Ch2), le premier canal d'accès (Ch1) reliant l'au moins une incision d'ouverture (Co) à la surface de lenticule postérieure (Lp) pour permettre l'accès d'un outil chirurgical à la surface de lenticule postérieure (Lp) à travers l'au moins une incision d'ouverture (Co), et le second canal d'accès (Ch2) reliant l'au moins une incision d'ouverture (Co) à la surface de lenticule antérieure (La) pour permettre l'accès de l'outil chirurgical à la surface de lenticule antérieure (La) à travers l'au moins une incision d'ouverture (Co), le premier canal d'accès (Ch1) et le second canal d'accès (Ch2) se chevauchant au moins partiellement depuis une vue de dessus de la cornée (20) .
